# EUROPEAN PATENT APPLICATION

(11) **EP 3 628 671 A1**
(43) Date of publication of application: **01.04.2020**
(21) Application number: 18197800.8
(22) Date of filing: 28.09.2018
(51) Int. Cl.: C07D 405/14, C08G 59/22

(54) **HIGH HEAT EPOXY COATING POWDERS, PROCESSES OF MAKING, AND USES THEREOF**

(71) Applicant: SABIC Global Technologies B.V., 4612 PX Bergen op Zoom (NL)
(72) Inventor: SISTA, Prakash, Mt. Vernon, IN 47620-9367 (US)
(74) Representative: Balder IP Law, S.L.

(57) **Abstract**

A coating powder including particles comprising a diepoxy phthalimidine of the formula (I): 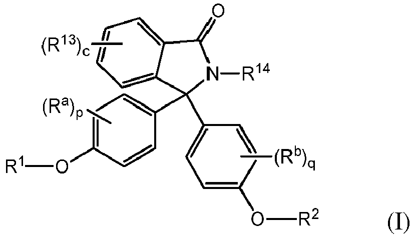 wherein R¹ and R² are each independently an epoxide-containing functional group, R^{a} and R^{b} at each occurrence are each independently halogen, C₁₋₁₂ alkyl, C₂₋₁₂ alkenyl, C₃₋₈ cycloalkyl, or C₁₋₁₂ alkoxy, R¹³ at each occurrence is independently a halogen or a C₁-C₆ alkyl group, p, q, and c are each independently 0 to 4, and R¹⁴ is a C₁₋₆ alkyl or phenyl; a curing agent co-curable with the diepoxy phthalimidine; optionally, a cure catalyst; and optionally, an auxiliary epoxy component co-curable with the diepoxy phthalimidine, the curing agent, or both.

## Description

### BACKGROUND

This disclosure relates to coating powders, and in particular to coating powders comprising high heat epoxies.

Coating powders are dry, finely-divided particulate compositions that are generally applied to substrates by electrostatic processes in which the powder particles are electrostatically charged and the substrate is earthed. The applied composition is then heated to melt and fuse the particles, and to cure the coating. The particles that do not adhere to the substrate can be recovered for reuse so that coating powders are economical in use of compositions. Because coating powders are generally free of added solvents and, in particular, do not use organic solvents, coating powders can accordingly be more economical and easier to manufacture, without the need to comply with regulations pertaining to use of organic solvents. Another advantage of coating powders is that they can be formulated to provide a variety of characteristics in the coating, for example low or high gloss.

In the powder coatings industry, high performance powder coatings are useful in functional applications such as oil and gas pipeline coatings and rebar coatings where chemical resistance and operational temperatures are important. In their cured form, epoxy polymers offer desirable properties including good adhesion to other materials, excellent resistance to corrosion and chemicals, high tensile strength, and good electrical resistance. However, there still remains a need in the art for coating powders that can form films have a high glass transition temperature (Tg). It would be a particular advantage if the high heat coating powders could provide a low gloss and a smooth homogenous surface. Also desirable is coating powders having a low cure temperature such that a wider variety of heat-sensitive substrates can be coated with the powder coating.

### SUMMARY

A coating powder is disclosed, comprising particles comprising a diepoxy phthalimidine of the formula (I): 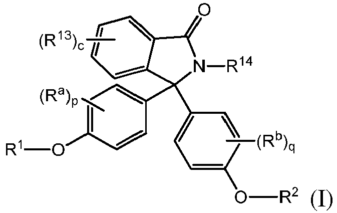 wherein R¹ and R² are each independently an epoxide-containing functional group, R^{a} and R^{b} at each occurrence are each independently halogen, C₁₋₁₂ alkyl, C₂₋₁₂ alkenyl, C₃₋₈ cycloalkyl, or C₁₋₁₂ alkoxy, R¹³ at each occurrence is independently a halogen or a C₁₋₆ alkyl group, p, q, and c are each independently 0 to 4, and R¹⁴ is a C₁₋₆ alkyl or phenyl; a curing agent; and optionally, an auxiliary epoxy component co-curable with the diepoxy phthalimidine.

A method of forming the coating powder is disclosed.

Also disclosed are a method of coating an article with the coating powder to form a powder coated article, and articles comprising the powder coating.

The invention is further illustrated by the following detailed description, examples, and claims.

### DETAILED DESCRIPTION

Disclosed herein are curable coating powders, particularly curable coating powders comprising diepoxy phthalimidine compounds, processes for making the curable coating powders, and articles coated with the curable coating powders. The compositions have desirable properties, including smooth, homogenous surfaces and an increase in hardness as measured per ASTM D3418.

The coating powder is curable, and comprises particles comprising a high heat diepoxy phthalimidine as described below, a curing agent, and optionally an auxiliary epoxy component co-curable with the diepoxy phthalimidine. The high heat a diepoxy phthalimidine is of formula (I): 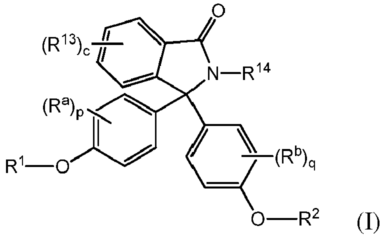 wherein R¹ and R² are each independently an epoxide-containing functional group, R^{a} and R^{b} at each occurrence are each independently halogen, C₁₋₁₂ alkyl, C₂₋₁₂ alkenyl, C₃₋₈ cycloalkyl, or C₁₋₁₂ alkoxy, R¹³ at each occurrence is independently a halogen or a C₁₋₆ alkyl group, p, q, and c are each independently 0 to 4, and R¹⁴ is a C₁₋₆ alkyl or phenyl; a curing agent co-curable with the diepoxy phthalimidine.

In an aspect, the R¹ and R² groups of the diepoxy phthalimidine of formula (I) are each independently of the formula 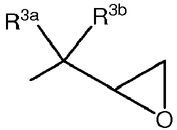 wherein R^{3a} and R^{3b} are each independently hydrogen or C₁₋₁₂ alkyl, R^{a} and R^{b} at each occurrence are each independently halogen, C₁₋₆ alkyl, C₂₋₆ alkenyl, C₃₋₆ cycloalkyl, or C₁₋₆ alkoxy, R¹³ at each occurrence is independently a halogen or a C₁₋₃ alkyl group, R¹⁴ is C₁₋₂ alkyl or phenyl, and p, q, and c are each independently 0 to 2.

In another aspect, the R¹ and R² groups of the diepoxy phthalimidine of formula (I) are each of the formula 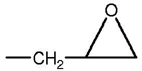 R^{a} and R^{b} are each independently halogen, C₁₋₃ alkyl, C₂₋₃ alkenyl, or C₁₋₃ alkoxy, R¹³ is a C₁₋₂ alkyl group, and p, q, and c are each independently 0 or 1.

In an aspect, the R¹ and R² groups of the the diepoxy phthalimidine of formula I at each occurrence are each independently 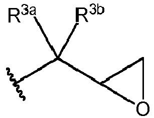 wherein R^{3a} and R^{3b} are each independently hydrogen or C₁₋₁₂ alkyl, R¹⁴ is C₁₋₂ alkyl or phenyl, and c, p, and q are each 0. In a preferred aspect, R^{3a} and R^{3b} are each independently hydrogen or C₁₋₃ alkyl and R¹⁴ is C₁₋₂ alkyl or phenyl.

In a more preferred aspect of formula I, the diepoxy phthalimidine is of formula (Ia) 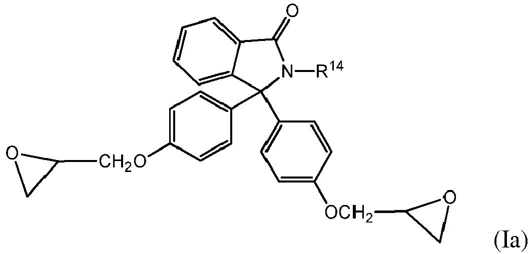 wherein R¹⁴ is methyl or phenyl. In still another preferred aspect of formula (Ia), R¹⁴ is phenyl.

The diepoxy phthalimidine compound can be prepared by methods described in, for example, WO2016/014536, from the corresponding bisphenol compound. The diepoxy phthalimidine can have a purity of greater than 60% or an epoxy equivalent weight of 1.0 to 1.5, or a combination of the purity and epoxy equivalent weight.

The diepoxy phthalimidine can have a purity of greater than 60%, preferably greater than 70%, preferably greater than 80%, preferably greater than 90%, preferably greater than 95%, preferably greater than 98%, or preferably greater than 99% as determined by high performance liquid chromatography, or an epoxy equivalent weight preferably 1.0 to 1.2, preferably 1.0 to 1.15, preferably 1.0 to 1.1, relative to the theoretical epoxy equivalent weight, or a combination of both the purity and the epoxy equivalent weight.

The diepoxy phthalimidine can have a metal impurity content of 3 ppm or less, 2 ppm or less, 1 ppm or less, 500 ppb or less, 400 ppb or less, 300 ppb or less, 200 ppb or less, or 100 ppb or less. The metal impurities can be iron, calcium, zinc, aluminum, or a combination thereof. The polyoxazolidone-phthalimidine oligomer can have an unknown impurities content of 0.1 wt.% or less.

The diepoxy phthalimidine can have a color APHA value of 40 or less, 35 or less, 30 or less, 25 or less, 20 or less, 19 or less, 18 or less, 17 or less, 16 or less, or 15 or less, as measured using test method ASTM D1209.

The coating powders are heat-curable, and further comprise a curing agent. The term "curing agent" as used herein encompasses compounds whose roles in curing epoxy compounds are variously described as those of a hardener, a hardening accelerator, a crosslinking agent, a curing catalyst, a curing co-catalyst, and a curing initiator, among others. Curing agents can have active hydrogen atoms that react with epoxy groups of the epoxy resin to form an extended or cross-linked resin. The active hydrogen atoms can be present in functional groups comprising primary or secondary amines, phenols, thiols, carboxylic acids, or carboxylic acid anhydrides. Curing agents can also function as an initiator for epoxy resin polymerization or as an accelerator for other curing agents.

Specific examples of curing agents for the powder coating include aliphatic amines, cycloaliphatic amines, aromatic amines, amine-functional adducts with epoxy resins, Mannich bases, polyamides, amidoamines, phenalkamines, dicyandiamide, dicyandiamide adducts, polycarboxylic acid-functional polyesters, carboxylic acid anhydrides, including aromatic dianhydrides and bicyclic anhydrides, amine-formaldehyde resins, phenol-formaldehyde resins, polysulfides, polymercaptans, imidazoles, imidazole adducts, Lewis acids such as boron trifluoride, diaryliodonium salts, or a combination thereof. In a preferred aspect, the curing agent is an aromatic amine, an aromatic dianhydride, a bicyclic anhydride, dicyandiamide, a dicyandiamide adduct, an imidazole, an imidazole adduct, or a combination thereof.

The curing agent can be an aromatic diamine. Examples of aromatic amine curing agents are 4,4'-diaminodiphenylmethane (DDM), 4,4'-diaminodiphenylsulfone (DDS), 4,4'-diamino diphenyl sulfone, 4,4'-methylenebis-(2,6-diethylaniline), 4,4'-methylenedianiline, diethyltoluenediamine, 4,4'-methylenebis-(2,6-dimethylaniline), m-phenylenediamine (MPDA), p-phenylenediamine, 2,4-bis(p-aminobenzyl)aniline, 3,5-diethyltoluene-2,4-diamine, 3,5-diethyltoluene-2,6-diamine, m-xylylenediamine, p-xylylenediamine, diethyl toluene diamine, or a combination thereof. Preferably the aromatic amine is 4,4'-diaminodiphenylmethane, 4,4'-diaminodiphenylsulfone (DDS), m-phenylenediamine, or a combination thereof.

The curing agent can be an aromatic dianhydride with the general structure 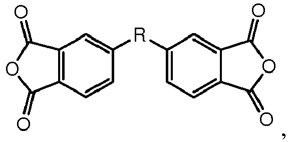 wherein R can be a single bond, 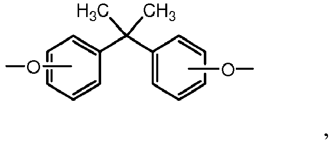 other bisphenols, -C(CF₃)₂-, - O-, or -C(=O)-. Examples of aromatic dianhydride curing agents include 4,4'-(4,4'-isopropylidenediphenoxy)bis-(phthalic anhydride) (CAS Reg. No. 38103-06-9), 4,4'-(hexafluoroisopropylidene)diphthalic anhydride (CAS Reg. No. 1107-00-2), 4,4'-oxydiphthalic anhydride (CAS Reg. No. 1823-59-2), benzophenone-3,3',4,4'-tetracarboxylic dianhydride (CAS Reg. No. 2421-28-5), and 3,3',4,4'-biphenyltetracarboxylic dianhydride (CAS Reg. No. 2420-87-3), and specifically compounds having the formulas below.

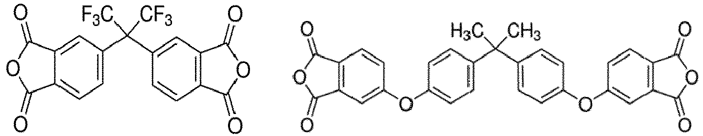

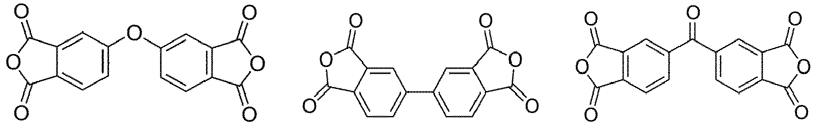

The curing agent can be a bicyclic anhydride, such as methyl-5-norbornene-2,3-dicarboxylic anhydride (CAS Reg. No. 25134-21-8) and cis-5-norbornene-endo-2,3-dicarboxylic anhydride (CAS Reg. No. 129-64-6), and specifically compounds having the formulas below.

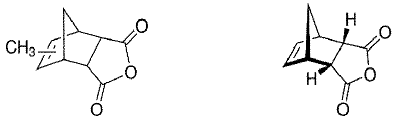

The curing agent can be a latent curing agent. Latent curing agents can be stably stored at room temperature (e.g., 21-25°C) in the presence of an epoxy resin and rapidly cure when exposed to heat, for example temperatures greater than 50°C. Non-limiting examples of latent curing agents are dicyandiamide, dicyandiamide adducts, boron trifluoride-amine complexes, organic acid hydrazides, imidazoles, and imidazole adducts. A combination of different latent curing agents can be used.

A suitable dicyandiamide curing agent is sold under the trade name DICY 100 from Alzchem. When the curing agent is dicyandiamide, an additional curing agent can be used, such as a tertiary amine, aromatic amine, imidazole, or a combination thereof. An adduct of the dicyandiamide and the amine can be used. In a preferred aspect, the curing agent is a dicyandiamide-imidazole adduct.

An imidazole can be used as a curing agent. Examples of suitable imidazoles have the formula 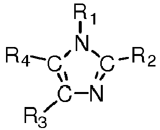 wherein R¹-R⁴ are each independently hydrogen, C₁₋₁₂ alkyl, C₆₋₁₈ aryl, C₇₋₁₈ arylalkylene, C₇-C₁₈ alkylarylene, or the like. Examples of suitable imidazoles include imidazole, 2-methyl imidazole, 4-methyl imidazole, 2,4-dimethyl imidazole, 2-ethyl-4-methyl imidazole, 2-methyl-4-ethyl imidazole, 4-butyl-5-ethyl imidazole, 2-cyclohexyl-4-methyl imidazole, 2-ethyl-4-phenyl imidazole, and 2-phenyl imidazole.

. Imidazoles can be used alone or in combination with an additional curing agent, for example anhydrides, dicyandiamide, polyhydric phenols, aromatic amines, and epoxy resins, such as bisphenol A epoxy resins. For example, an imidazole carboxylate, an epoxy-imidazole adduct, a metal salt-imidazole complex compound, or an imidazole that has been reacted with an acidic substance can be used as a curing agent.

In a preferred aspect, an imidazole adducts can be used, such as an adduct of the imidazole with an anhydride, dicyandiamide, polyhydric phenol, aromatic amine, or epoxy resin. Mixtures of imidazole adducts can be used. The imidazoles tend to be insoluble in epoxy resins. Thus, adducting an imidazole to an epoxy resin increases its compatibility with the epoxy system. A suitable epoxy-imidazole adduct is a bisphenol A epoxy- imidazole adduct, available commercially as, for example, EPIKURE P-101. Other commercially available imidazole adduct includes EPIKURE Curing Agent P-101, P-103, P-104, and P-108 from Momentive and the phenolic imidazole adduct curing agent available as EPIKURE Curing Agent P-202 from Momentive. The curing agent can be a dicyandiamide-imidazole adduct. A suitable dicyandiamide imidazole adduct is sold under the tradename EPIKURE P-108.

In an aspect, the curing agent is 44,4'-diaminodiphenylmethane, 4,4'-diaminodiphenylsulfone, m-phenylenediamine, methyl-5-norbornene-2,3-dicarboxylic anhydride, dicyandiamide, an amide adduct of dicyandiamide, imidazole, 2-methyl imidazole, 4-methyl imidazole, 2,4-dimethyl imidazole, 2-methyl-4-ethyl imidazole, 2-ethyl-4-methyl imidazole, 2,4-diethyl imidazole, a dicyandiamide-imidazole adduct, an epoxy resin-imidazole adduct, or a combination thereof.

In a more preferred aspect, the curing agent is 4,4'-diamino diphenyl sulfone , dicyandiamide, imidazole, 2-ethyl-4-methyl imidazole, a dicyandiamide-imidazole adduct, a bisphenol A epoxy-imidazole adduct, or a combination thereof.

The curing agent is used in an amount effective to cure the coating powder. The specific amount of curing agent used will depend on the identity of the high heat epoxy and the curing agent, among other factors, and can be readily determined by the skilled person. For example, when the curing agent is an aromatic diamine compound, it can be used in an amount of 1 to 35 parts by weight, based on the total parts by weight of the epoxy resin, including any auxiliary co-curable epoxy resin as described below.

In some aspects, the coating powder further comprises an auxiliary epoxy component co-curable with the diepoxy phthalimidine. In an aspect, the auxiliary epoxy is an aliphatic epoxy, cycloaliphatic epoxy, aromatic epoxy, multi-aromatic epoxy such a bisphenol A epoxy or bisphenol-F epoxy, phenol novolac epoxy, cresol-novolac epoxy, biphenyl epoxy, triglycidyl p-aminophenol, tetraglycidyl diamino diphenyl methane, a polyfunctional epoxy, a naphthalene epoxy, divinylbenzene dioxide, 2-glycidylphenylglycidyl ether, a dicyclopentadiene epoxy, or a combination thereof. Any of the foregoing epoxies can be a compound (e.g., bisphenol A diglycidylether), or oligomeric or polymeric (e.g., containing more than one bisphenol A unit). Any of the foregoing epoxies can be polyfunctional, i.e., contain two or more epoxy groups.

In an aspect the auxiliary epoxy is a bisphenol A diglycidylether, a bisphenol F diglycidylether, a neopentylglycol diglycidyl ether, a 3,4-epoxycyclohexylmethyl-3,4-epoxycyclohexane carboxylate, a *N,N*-diglycidyl-4-glycidyloxyaniline, an N,N,N',N'-tetraglycidyl-4,4'-diaminodiphenylmethane, or a combination thereof.

In a preferred aspect, the auxiliary epoxy component is a solid, which means that the auxiliary epoxy component is solid or semi-solid at ambient temperature (i.e., 23°C) and one atmosphere pressure. In some embodiments, the auxiliary epoxy component has a softening point of 25°C to 150°C. Softening points can be estimated by some arbitrary methods. For example, above the glass transition temperature as determined by differential scanning calorimetry (DSC), dynamic mechanical analysis (DMA) or ring and ball test methods as described in ASTM E28-67, ASTM E28-99, ASTM D36, ASTM D6493-11, and ISO 4625. Solid auxiliary epoxies can be derived from liquid epoxy resin and a bisphenol, for example bisphenol-A. One of ordinary skill in the art would understand that chemical structural modifications can be made to known liquid epoxies (at ambient temperature and pressure) to render them as solids at ambient temperature and pressure and to increase the softening temperature or the Tg. For example, a bisphenol A diglycidyl epoxy can be structurally modified to provide the material commercially available as D.E.R.TM 6510, a solid at ambient temperature and pressure.

Suitable auxiliary epoxy solid compounds that are commercially available include for example D.E.R.™ 660, D.E.R.™ 6508, D.E.R.™ 671, D.E.R.™ 642U-20, D.E.R.™ 661, D.E.R.™ 642U, D.E.R.™ 6116, D.E.R.™ 662E, D.E.R.™ 6225, D.E.R.™ 692, D.E.R.™ 692H, D.E.R.™ 692HB, D.E.R.™ 6224, D.E.R.™ 662UH, D.E.R.™ 6615, D.E.R.™ 663UE, D.E.R.™ 663U, D.E.R.™ 672U-20, D.E.R.™ 663-A25, D.E.R.™, 672U, D.E.R.™ 663UK, D.E.R.™ 6330-A10, D.E.R.™ 640, D.E.R.™ 664UE, D.E.R.™ 664U, D.E.R.™ 6155, D.E.R.™ 666E, D.E.R.™ 6670, D.E.R.™ 667-20, D.E.R.™ 667E, D.E.R.™ 668-20, D.E.R.™ 669E, D.E.R.™ 669-20, D.E.R. 6510-HT, D.E.R. 6508, epoxidized cyclohexane tetraphenol (eCHTP), D.E.N. 438, D.E.N. 439; resins commercially available under the tradename "TACTIX" such as TACTIX®556, TACTIX®742, TACTIX®756; and ARALDITE MY 720; or a combination thereof.

The auxiliary epoxy component can be present from 1 to 99 wt%, or 5 to 95 wt%, or 50 to 90 wt%, or 5 to 50 wt%, based on the total combined weight of the epoxies.

The coating powder can comprise an additive, wherein the additive is a flow control agent, dry flow agent, antioxidant, pigment, dye, optical brightener, extender, heat stabilizer, light stabilizer, ultraviolet light stabilizer, ultraviolet light-absorbing compound, near infrared light-absorbing compound, infrared light-absorbing compound, plasticizer, lubricant, antistatic agent, anti-fog agent, antimicrobial agent, radiation stabilizer, flame retardant, anti-drip agent, fragrance, or a combination thereof. Any additive is used in an amount generally known to be effective, which can be from 0.001 to 10 parts by weight, per 100 parts by weight of the total amount of epoxy resin in the coating powder For example, the total amount of the additives (other than any filler or pigment) can be 0.01 to 20 parts by weight, or 1 to 10 parts by weight, per 100 parts by weight of the total amount of epoxy resin in the coating powder.

Flow control agents, sometimes called leveling agents, are useful to promote the formation of a continuous coating. Suitable flow control agents include polyacrylic esters, non-ionic fluorinated alkyl ester surfactants, non-ionic alkylarylene polyether alcohols, silicones, and the like, and combinations comprising at least one of the foregoing flow control agents. Flow control agents are generally liquids that have been converted to powder form by absorption onto silica-type materials. A preferred flow control agent is sold under the tradename RESIFLOW® P-67 acrylic resin by Estron Chemical, Inc., which is a 2-propenoic acid, ethyl ester polymer. Another preferred flow control agent is benzoin, which is a 2-hydroxy-1,2-diphenylethanone crystalline solid that is believed to keep the molten coating open for a suitable time to allow outgassing to occur prior to the formation of the hard set film. When present, the flow control agent can be used at an amount of 1 part by weight to 5 parts by weight, per 100 parts by weight of epoxy resin.

Suitable dry flow agents include fumed silica (for example that sold under the tradename CAB-O-SIL® by Cabot Corporation) and fumed alumina, for example that sold under the tradename Aluminum Oxide C by Degussa Corporation). When present, the dry flow agent can be used in an amount of 0.05 to 0.5 parts by weight, per 100 parts by weight of the total amount of epoxy resin in the coating powder.

Pigments can be used to adjust color and opacity. Suitable pigments include, for example, titanium dioxide, carbon black, phthalocyanine blue, phthalocyanine green, quinacridone red, perylene red, isoindolone yellow, dioxazine violet, scarlet 3B lake, red 188 azo red, azo pigment yellow 83, iron oxide pigments, and the like. When present, the pigment can be used in an amount of up to 100 parts by weight per 100 parts by weight of the total amount of epoxy resin in the coating powder.

The coating powders can contain a filler. Suitable fillers include calcium carbonate, barium sulfate, dolomite, wollastonite, talc, mica, and the like. When present, the filler can be used in an amount up to 120 parts by weight per 100 parts by weight of the total amount of epoxy resin in the coating powder. Within this range, a filler amount of at least 10 parts by weight is preferred. Also within this range, a filler amount of up to 80 parts by weight is preferred.

The coating powder can comprise an antioxidant. Antioxidants prevent discoloration of the coatings. Suitable antioxidants include, for example, sodium hypophosphite, tris-(2,4-di-t-butyl phenyl) phosphite (available as IRGAFOS 168 from Ciba-Geigy), calcium bis([monoethyl(3,5-di-t-butyl-4-hydroxybenzyl)phosphonate] (available as IRGANOX 1425 from Ciba-Geigy), and the like. Mixtures of antioxidants can be used. The sodium hypophosphite can also act as a buffer against the action of trace amounts of chlorine released by epichlorohydrin residues in the epoxy resins. When present, antioxidants can be used in an amount of 0.5 to 2.0 parts by weight per 100 parts by weight of the total amount of epoxy resin in the coating powder.

The coating powder can exclude any solvent. In this context it will be understood that "solvents" does not include liquid aromatic epoxy polymers. In these embodiments, excluded solvents include, for example, C₃₋₈ ketones, C₄₋₈ N,N-dialkylamides, C₆₋₁₂ aromatic hydrocarbons, C₄₋₁₆ dialkyl ethers, C₃₋₆ alkyl alkanoates, and C₂₋₆ alkyl cyanides. The exclusion of solvents is desirable because they are unnecessary to dissolve the epoxy compound and because their presence reduces the glass transition temperature of the solid composition. The solid compositions can include a solvent to prepare homogeneous coating powder composition and then the solvent can be removed.

Preferred methods for forming the powder coating include melt mixing, in which the dry ingredients of the composition for forming the coating powder are weighed into a batch mixer and are mixed with a medium intensity horizontal plowmixer or a lesser intensity tumble mixer. Mixing times can be from 1 to 3 minutes for the high intensity mixers to 30 to 60 minutes for the tumble mixers. The premix can then be further mixed and compounded as the resin is melted in either a single screw or a twin screw extruder for 0.5 to 1 minute, generally at 50 to 100°C, with control of the extruder temperature to minimize any curing and gelation from taking place in the extruder. The aforesaid extruder temperatures are lower than the typical cure temperatures of the powder coatings, which can begin initial curing at temperatures starting at 100°C.

After extrusion, the coating powder compositions are usually in chip form. After cooling, the coating powder compositions are ground together in a mill, such as a Brinkman mill or a Bantam hammer mill, to achieve the desired particle size. Particle size determines the coarseness or fineness of the texture of the coating powder on the substrate. Generally, the particle size is from 60 mesh (for coarse) to 200 mesh (for fine), depending upon the desired texture. Scalping at 100 mesh is typical to remove coarse particles. In some aspects, the coating powder can have an average particle size of 10 to 100 micrometers. Average particle size is typically 20 to 80 micrometers. There is typically about 10-15 wt% of particles below 11 micrometers and 0-4 wt% of particles above 88 micrometers. Particle size can be determined by laser diffraction techniques, using, e.g., a Malvern particle size analyzer.

The coating powders can be shelf stable, and do not separate into individual components during typical powder coating application.

A method for manufacturing a powder coated article comprises applying the coating powder to a surface of the article; and the applied coating powder to melt and fuse the particles to form a films and cure the film. The heating can be by applying the coating powder to a surface of the article; then heating the coated article to melt and fuse the particles to form a film and cure the film. Another method for coating an article comprises heating the the article and applying the coating powder to a surface of the heated article. Of course, a combination of the two methods can be used, where the article is pre-heated, and then placed in heated environment to further melt and fuse the particles to form a film and cure the film.

The coating powder can be applied to an article (i.e, a substrate) by conventional means, including electrostatic fluidized beds, electrostatic spray guns, triboelectric guns, and the like, in which the powder coating particles are electrostatically charged and the substrate is grounded or oppositely charged. The substrate is heated (and can optionally be pre-heated prior to application), to aid the melt, flow, and coalescence of the particles to form a smooth, continuous film. Coating powders are generally applied to achieve a coating thickness of 1.0 mil (0.0245 millimeters, "mm") to 25 mils (0.102 mm), preferably least 1.5 to 4 mils (0.038 to 0.1 mm). For decorative finishes, film thicknesses as low as 20 micrometers should be mentioned, but it is more usual for the film thickness to fall within the range of 25 to 120 micrometers, with common ranges being 30 to 80 micrometers for some applications, and 60 to 120 micrometers or, more preferably, 60 to 100 micrometers for other applications, while film thicknesses of 80 to 150 micrometers are less common.

The coating film is then cured to a thermoset state without degrading the substrate. Heating can be performed in infrared, convection ovens, or a combination of both, although infrared ovens are preferred. Time and temperature of the final cure will vary somewhat depending on the film-forming systems employed and on the conditions of use. The curing temperature can be 100 to 300°C. One advantage of the curable compositions is their ability to produce matte and low gloss finishes at lower curing temperatures. In an aspect, the curing temperature is 150°C to 250°C. Cure times can be 2 seconds to up to 30 minutes, preferably up to 20 minutes, more preferably up to 10 minutes. Advantageously, the cure time can be less than 10 minutes, for example less than 30 seconds, less than 1 minute, less than 2 minutes, less than 3 minutes, less than 4 minutes, less than 5 minutes, less than 6 minutes, less than 7 minutes, less than 8 minutes, or less than 9 minutes. Regardless of cure time and temperatures used, provided that the components of the coating powder composition have been sufficiently melted before curing, the powder coatings generated on the substrates can have a visually consistent appearance.

The coating powder can be used in coating glass, ceramics, high temperature polymers, and graphite-filled composites, as well as metallic substrates such as steel and aluminum. The coating powder is particularly useful for coating parts as further described below.

Advantageously, the powder coating can have a glass transition temperature (2) higher than a powder coating of the same composition but with a bisphenol A-based epoxy instead of the diepoxy phthalimidine. In other words, substitution of all or a part of a commercially available bisphenol A epoxy with the diepoxy phthalimidine in a coating powder composition can result in a powder coating having a higher glass transition temperature than if the diepoxy phthalimidine were not present. This feature is particularly advantageous where articles using the powder coatings are in higher heat environments, for example in oil or gas extraction, or reaction vessels.

Also dvantageously, the powder coating can have a greater hardness than a powder coating of the same composition but with a bisphenol A-based epoxy instead of the diepoxy phthalimidine. In other words, substitution of all or a part of a commercially available bisphenol A epoxy with the diepoxy phthalimidine in a coating powder composition can result in a powder coating having a higher hardness than if the diepoxy phthalimidine were not present.

The powder coating can have a a glass transition temperature greater than 120°C, greater than 140°C, greater than 150°C, greater than 180°C, preferably greater than 190°C. For example, the powder coating can have a Tg of greater than or equal to 120°C , for example up to 250°C, as measured by differential scanning calorimetry (DSC). In an embodiment, a cured sample of the coating powder has a Tg of 120 to 259°C as measured by DSC, or 180 to 250°C as measured by DSC. In preferred embodiments, a cured sample of the coating powder has a Tg of 190 to 250°C, as measured by DSC.

The powder coating can exhibit a color difference value (ΔE) of less than or equal to 1.0 after 252 kJ/m2 of exposure, preferably less than or equal to 0.7 after 252 kJ/m2 of exposure, more preferably 0.5 after 252 kJ/m2 of exposure as measured according to ASTM 2244.

The powder coating can exhibit a gloss of 75 to 120, preferably 80 to 110 as measured according to ASTM D-523.

The coating powders can be used in a variety of applications, such as composites for aerospace applications, the electronics industry, oil and gas pipeline coatings, and rebar coatings. The coating powders can be used in a variety of forms for various purposes, including the manufacture of a composite (e.g., composite materials such as those using carbon fiber and fiberglass reinforcements), a layer, a coating, an encapsulant, an adhesive, a sealant, a sizing resin, a prepreg, or a combination thereof.

Applications for the coating powders comprising a diepoxy phthalimidine compound include, for example, coatings for acid bath containers; neutralization tanks; aircraft components; bridge beams; bridge deckings; electrolytic cells; exhaust stacks; scrubbers; sporting equipment; stair cases; walkways; automobile exterior panels such as hoods and trunk lids; floor pans; air scoops; pipes and ducts, including heater ducts; industrial fans, fan housings, and blowers; industrial mixers; boat hulls and decks; marine terminal fenders; tiles and coatings; building panels; business machine housings; trays, including cable trays; concrete modifiers; dishwasher and refrigerator parts; electrical encapsulants; electrical panels; tanks, including electrorefining tanks, water softener tanks, fuel tanks, and various filament-wound tanks and tank linings; furniture; garage doors; gratings; protective body gear; luggage; outdoor motor vehicles; pressure tanks; printed circuit boards; optical waveguides; radomes; railings; railroad parts such as tank cars; hopper car covers; car doors; truck bed liners; satellite dishes; signs; solar energy panels; telephone switchgear housings; tractor parts; transformer covers; truck parts such as fenders, hoods, bodies, cabs, and beds; insulation for rotating machines including ground insulation, turn insulation, and phase separation insulation; commutators; core insulation and cords and lacing tape; drive shaft couplings; propeller blades; missile components; rocket motor cases; wing sections; sucker rods; fuselage sections; wing skins and flarings; engine narcelles; cargo doors; tennis racquets; golf club shafts; fishing rods; skis and ski poles; bicycle parts; transverse leaf springs; pumps, such as automotive smog pumps; electrical components such as printed wiring boards; scrubbing towers; swimming pools, swimming pool slides, hot-tubs, and saunas; drive shafts for under the hood applications; marine tooling and composites; heat shields; submarine hulls; laminated trim; industrial metal forming dies; aircraft stretch block and hammer forms; vacuum molding tools; flooring, including flooring for production and assembly areas, clean rooms, machine shops, control rooms, laboratories, parking garages, freezers, coolers, and outdoor loading docks; for decorative flooring; expansion joints for bridges; machinery rails; metal dowels; bolts and posts; and numerous other applications.

The compositions and methods described herein are further illustrated by the following non-limiting examples.

### EXAMPLES

The materials shown in Table 1 were used.

**Table 1.**

| Component | Description | Source |
|---|---|---|
| Epoxy resin-1 | Commercially available solid epoxy resin derived from liquid epoxy resin and bisphenol-A, EEW = 875 to 975, available as KD-214C | Kukdo |
| Epoxy resin-2 | Commercially available solid epoxy resin, EEW = 400-450, available as DER 6510 HT | Olin Chemical |
| PPPBP-epoxy | 1,1-bis(4-epoxyphenyl)-N-phenylphthalimidine | SABIC |
| Ep-P108 | Dicyandiamide-imidazole adduct curing agent, available as EPIKURE P-108 | Hexion |
| Ep-P101 | 2-Methyl-imidazole adduct with bisphenol-A epoxy resin, available as EPIKURE P-101 | Hexion |
| Dicy | Dicyandiamide curing agent, available as DICY 100 | Alzchem |
| 2E4MZ | 2-Ethyl-4-methylimidazole curing agent, available as Curezol 2E4MZ | Evonik |
| Filler | Wollastonite, median particle size = 3 micrometer BET surface area = 4.1 m²/g, available as NYAD M9000 | Imerys |
| 4,4'-DDS | 4,4-diaminodiphenyl sulfone curing agent, micronized | |
| Flow promoter | Liquid acrylic polymer adsorbed onto silica, available as RESIFLOW P-67 | Estron Chemical |
| Benzoin | Degassing agent | Estron Chemical |
| TiO₂ | Pigment, available as TiPure R960 | Chemours |

The following test methods were used.

Particle size was determined by Malvern particle analyzer. The particle size distribution was measured in water using laser diffraction (Mastersizer 3000 from Malvern). The dry powder was made into a slurry with DI water containing surfactant and sonicated for five minutes. The slurry was added to the Malvern reservoir, which contained DI water. The volume-based particle size distribution and number-based particle size distribution were measured.

Pellet flow was determined by pressing the powder into the form of a pellet and sticking it to a metal plate and placing it in a curing oven at 60° angle and cured according to the cure protocol mentioned. The flow distance is measured as the distance from the initial position of the pellet to the end of the flow.

Gel temperature was determined by chemorheology measurements. Rheological measurements were performed using a disposable 8 mm plate with 10% strain at a fixed gap of 1 mm using an ARES G2 strain controller rheometer. In this case the plates were pre-heated to 120 °C and were ramped to 200 °C at 20 °C/min.

Gloss at 60° was measured according to ASTM D-523 using a TQC PolyGloss 0030 gloss meter.

Color was measured according to ASTM D2244 using a Minolta hand-held colorimeter using the CIELab color scale and a D65 (horizon sunset) illuminant..

Color stability (ΔE) was determined by the difference in color between the colors of the cured samples and overbaked samples.

Impact resistance was measured according to ASTM D-2794. A 4 lb. rod was lifted to about 40 inches from the base of the panel containing the coating and dropped on the top of the panel and the back of the panel to obtain impact resistance in the forward and reverse directions.

Solvent resistance was determined by wetting a cloth with methyl ethyl ketone (MEK) and rubbing the coating with 100 double rubs. This was according to ASTM D-5402.

Adhesion was determined by scratching the coating with a crosshatch and measuring the percentage of the area of the coating that gets peeled off during the scratch test and an adhesion code ranging from 0B to 5B was assigned according to ASTM D-3359B.

Pencil hardness was determined by scratching the coating with a pencil that has a lead of a certain hardness. The hardness rating is determined by the maximum hardness of the lead at which a scratch does not appear. This test was performed according to ASTM D3363.

Glass transition temperature (Tg) was determined by TA Q1000 DSC or TA 2920 M-DS instrument (TA Instruments). In a typical procedure, a sample (10 to 20 mg) was heated from 40°C to 325°C (20°C/min), held at 325°C for 1 min, cooled back to 40°C (20°C /min), then held at 40°C for 1 min, and the above heating/cooling cycle was repeated. The second heating cycle is usually used to obtain the Tg.

The coating powders were prepared as follows. PPPBP-epoxy resin was mixed with all the other components of the formulation in a blender, the powder was then extruded, the ribbons were broken and ground to a fine powder, and the powder was passed through a sieve to obtain a powder of less than 100 µm particle size.

Films comprising the cured coating powders were made as follows. Coating powder compositions were applied to a metal substrate using an electospray gun. The powder-coated metal substrates were subsequently placed in an oven maintained at a constant temperature in order to obtain a cured film.

Formulations and physical characteristics are shown in Table 2. Comparative Examples 1, 5, and 8 (CEx 1, 5, and 8) are based on commercially available epoxy resins with no PPPB-epoxy.

**Table 2.**

| Component (g) | CEx1 | Ex2 | Ex3 | Ex4 | CEx5 | Ex6 | Ex7 | CEx8 | Ex9 | CEx10 | Ex11 |
|---|---|---|---|---|---|---|---|---|---|---|---|
| Epoxy resin-1 | 325.4 | 244 | 162.7 | 244.0 | | | | | | 298.5 g of CEx8 | 298.5 g of Ex9 |
| Epoxy resin-2 | | | | | 375.4 | 187.7 | | 298.9 | | | |
| PPPBP-epoxy (pre-melted) | | 70.3 | 140.7 | | | 184.1 | 368.3 | | 267.1 | | |
| PPPBP-epoxy (powder) | | | | 70.3 | | | | | | | |
| DCnD-Im | 17.1 | 28.1 | 39.1 | 28.1 | | | | | | | |
| P-Im | | | | | 6.0 | 6.0 | 6.0 | | | | |
| 2,4-EMI | | | | | | | | | | 1.5 | 1 |
| DCnD | | | | | 11.6 | 15.2 | 18.7 | | | | |
| Im | | | | | 6.8 | 6.8 | 6.8 | | | | |
| Filler | | | | | 97.6 | 97.6 | 97.6 | | | | |
| 4,4'-DDS | | | | | | | | 43.6 | 63.9 | | |
| Flow promoter | 5 | 5.0 | 5.0 | 5.0 | | | | | | | |
| Benzoin | 2.5 | 2.5 | 2.5 | 2.5 | | | | 2.5 | 2.4 | | |
| TiO₂ | 150 | 150 | 150 | 150 | | | | 150.0 | 141.1 | | |
| Properties | CEx1 | Ex2 | Ex3 | Ex4 | CEx5 | Ex6 | Ex7 | CEx8 | Ex9 | CEx10 | Ex11 |
| % of PPPBP-epoxy | 0 | 20 | 50 | 20 | 0 | 50 | 100 | 0 | 100 | 0 | 100 |
| Particle size | | | | | | | | | | | |
| D10 | 16.5 | 12.8 | 10.6 | 13.2 | 7.53 | 8.39 | 2.98 | | | 11.1 | 5.59 |
| D50 | 53.4 | 46.6 | 43.2 | 45.5 | 38.6 | 52.3 | 25.8 | | | 41 | 28.1 |
| D90 | 108 | 98.1 | 96.6 | 97.7 | 94.2 | 116 | 82.3 | | | 92.2 | 77.8 |

| Cure conditions | | | | | | | | | | | |
|---|---|---|---|---|---|---|---|---|---|---|---|
| Time (min) | 10 | 10 | 10 | 10 | 3 | 3 | 3 | | | 3 | 3 |
| Temp. (°C) | 200 | 200 | 200 | 200 | 240 | 240 | 240 | | | 240 | 240 |
| Pellet Flow (mm) | 75 | 48 | 35 | 46 | 44 | 58 | 90 | | | N/A | N/A |
| Gel Temp. (°C) | 169.5 | 155.6 | 152.5 | 156.1 | 148.4 | >150 | >146 | | | 136.2 | N/A |
| Gloss at 60° | 104 | 98.8 | 80.4 | 91.3 | 100 | 107 | 107 | | | 90.9 | 98.5 |
| Impact resist. | | | | | | | | | | | |
| Forward | 160 | 60 | <20 | 40 | 40 | <20 | <20 | | | 80 | <20 |
| Reverse | 160 | 20 | <20 | <20 | <20 | <20 | 20 | | | 80 | <20 |
| Solvent resist. | 100DR | 100DR | 100DR | 100DR | 100DR | 100DR | 100DR | | | 100DR | 100DR |
| Adhesion | 5B | 4B | 4B | 4B | 2B | 2B | 1B | | | 1B | 1B |
| Pencil hardness | 3H | 6H+ | 6H+ | 6H+ | 6H+ | 6H+ | 6H+ | | | 6H+ | 6H+ |
| Tg (°C) | 108 | 127 | 152 | 126 | 141 | 172 | 200 | | | 140 | 194 |

| Initial Color | | | | | | | | | | | |
|---|---|---|---|---|---|---|---|---|---|---|---|
| L | 96.01 | 95.89 | 95.51 | 95.96 | 61.47 | 62.79 | 61.7 | | | 86.18 | 89.44 |
| a | -0.82 | -0.71 | -0.82 | -0.56 | -0.1 | 0.17 | 0.14 | | | 2.38 | -0.05 |
| b | 4.15 | 4.29 | 4.57 | 4.53 | 15.67 | 11.93 | 10.8 | | | 19.66 | 18.9 |

| Overbake Color | | | | | | | | | | | |
|---|---|---|---|---|---|---|---|---|---|---|---|
| L | 93.58 | 92.86 | 91.91 | 92.57 | 56.14 | 58.25 | 58.63 | | | 81.9 | 88.2 |
| a | 0.14 | 0.62 | 0.95 | 0.84 | 2.52 | 1.34 | 0.64 | | | 2.19 | 0.21 |
| b | 9.36 | 11.07 | 13.24 | 11.84 | 24.56 | 19.49 | 14.76 | | | 23.97 | 20.78 |
| ΔE | 5.83 | 7.54 | 9.55 | 8.18 | 10.69 | 8.9 | 5.03 | | | 6.07 | 2.26 |

The data in Table 2 show that blends of PPPBP-epoxy with BPA-epoxy (Ex2, Ex3, and Ex4) demonstrate reduced gloss compared to BPA-epoxy alone (CEx1). The film comprising PPPBP-epoxy only (Ex7) demonstrates higher gloss than the high temperature commercial epoxy (CEx5).

The data in Table 2 show that blends of PPPBP-epoxy with BPA-epoxy (Ex2, Ex3, and Ex4) demonstrate lower color stability than BPA-epoxy alone (CEx1). The color stability of the high temperature commercial epoxy (CEx5) is improved by the addition of PPP-epoxy (Ex6, Ex 10, and Ex11).

The data regarding the films in Table 2 show that the examples containing the PPPBP-epoxy coating (Ex 2-4) demonstrated significant enhancement in thermal properties and color stability compared to those of a commercially available high temperature epoxy (CEx5) HH coating powder grade.

Addition of PPP-epoxy to BPA-epoxy based formulation leads to significant improvement in hardness of the coating, although a reduction in impact resistance, adhesion, and color stability can also be seen.

The invention is further illustrated by the following non-limiting aspects.
Aspect 1: A curable powder coating, comprising particles comprising a diepoxy phthalimidine of formula (I): 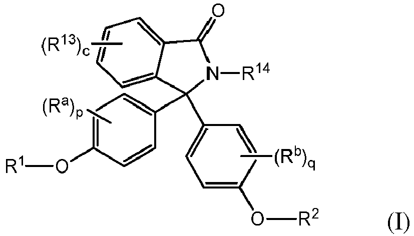 wherein R¹ and R² are each independently an epoxide-containing functional group, R^{a} and R^{b} at each occurrence are each independently halogen, C₁₋₁₂ alkyl, C₂₋₁₂ alkenyl, C₃₋₈ cycloalkyl, or C₁₋₁₂ alkoxy, R¹³ at each occurrence is independently a halogen or a C₁-C₆ alkyl group, p, q, and c are each independently 0 to 4, and R¹⁴ is a C₁₋₆ alkyl or phenyl; a curing agent; and optionally, an auxiliary epoxy component co-curable with the diepoxy phthalimidine.
Aspect 2: The coating powder of aspect 1, R¹ and R² are each independently of the formula 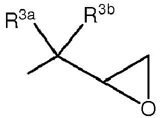 wherein R^{3a} and R^{3b} are each independently hydrogen or C₁₋₁₂ alkyl, preferably wherein R^{3a} and R^{3b} are each hydrogen, R^{a} and R^{b} at each occurrence are each independently halogen, C₁₋₆ alkyl, C₂₋₆ alkenyl, C₃₋₆ cycloalkyl, or C₁₋₆ alkoxy, preferably halogen, C₁₋₆ alkyl, C₂₋₆ alkenyl, C₃₋₆ cycloalkyl, or C₁₋₆ alkoxy, R¹³ at each occurrence is independently a halogen or a C₁₋₃ alkyl group, preferably a C₁₋₂ alkyl group, R¹⁴ is C₁₋₂ alkyl or phenyl, and p, q, and c are each independently 0 to 2.
Aspect 3: The coating powder of any one or more of the preceding aspects, wherein R¹ and R² at each occurrence are each independently 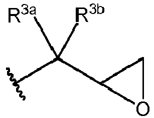 wherein R^{3a} and R^{3b} are each independently hydrogen or C₁₋₁₂ alkyl, preferably hydrogen; R¹⁴ is C₁₋₂ alkyl or phenyl, , preferably methyl or phenyl, and c, p, and q are each 0.
Aspect 4: The coating powder of any one or more of the preceding aspects, wherein the diepoxy phthalimidine is of the formula 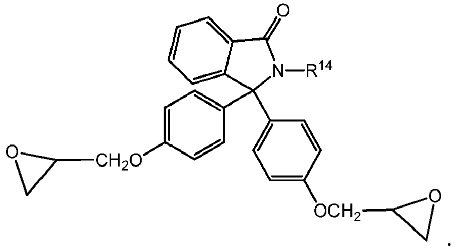 wherein R¹⁴ is methyl or phenyl, preferably wherein R¹⁴ is phenyl.
Aspect 5: The coating powder of any one or more of the preceding aspects, wherein diepoxy phthalimidine has a purity of greater than 60%, preferably greater than 70%, preferably greater than 80%, preferably greater than 90%, preferably greater than 95%, preferably greater than 98%, or preferably greater than 99% as determined by high performance liquid chromatography, or an epoxy equivalent weight of 1.0 to 1.5, preferably 1.0 to 1.2, preferably 1.0 to 1.15, preferably 1.0 to 1.1, relative to the theoretical epoxy equivalent weight, or a combination of the purity and epoxy equivalent weight.
Aspect 6: The coating powder of claim of any one of the preceding aspects, wherein the curing agent is an aliphatic amine, a cycloaliphatic amine, an aromatic amine, an amine-functional adduct with an epoxy resin, a Mannich bases, a polyamide, an amidoamine, a phenalkamine, a dicyandiamide, a polycarboxylic acid-functional polyester, a carboxylic acid anhydride an, amine-formaldehyde resin, a phenol-formaldehyde resin, a polysulfides, a polymercaptan, an imidazole, an imidazole adduct, a Lewis acid, a diaryliodonium salt, or a combination thereof; preferably wherein the curing agent is an aromatic amine, an aromatic dianhydride, a bicyclic anhydride, dicyandiamide, a dicyandiamide adduct, an imidazole, an imidazole adduct, or a combination thereof; preferably wherein the curing agent is 4,4'-diaminodiphenylmethane, 4,4'-diaminodiphenylsulfone, m-phenylenediamine, methyl-5-norbornene-2,3-dicarboxylic anhydride, dicyandiamide, an amide adduct of dicyandiamide, imidazole, 2-methyl imidazole, 4-methyl imidazole, 2,4-dimethyl imidazole, 2-methyl-4-ethyl imidazole, 2-ethyl-4-methyl imidazole, 2,4-diethyl imidazole, a dicyandiamide-imidazole adduct, an epoxy resin-imidazole adduct, or a combination thereof; more preferably wherein the curing agent is 4,4'-diamino diphenyl sulfone , dicyandiamide, imidazole, 2-ethyl-4-methyl imidazole, a dicyandiamide-imidazole adduct, bisphenol A epoxy-imidazole adduct, or a combination thereof.
Aspect 7: The coating powder of claim of any one or more of the preceding aspects, further comprising the auxiliary epoxy component co-curable with the diepoxy phthalimidine, wherein the auxiliary epoxy component is an aliphatic epoxy, cycloaliphatic epoxy, aromatic epoxy, multiaromatic epoxy, phenol novolac epoxy, cresol-novolac epoxy, biphenyl epoxy, triglycidyl p-aminophenol, tetraglycidyl diaminodiphenyl methane, naphthalene epoxy, divinylbenzene dioxide, 2-glycidylphenylglycidyl ether, dicyclopentadiene epoxy, or a combination thereof; preferably wherein the auxiliary component is a solid or semi-solid at 23°C.
Aspect 8: The composition of aspect 7, comprising 1 to 99 wt%, or 5 to 95 wt%, or 50 to 90 wt%, or 5 to 50 wt% of the auxiliary epoxy component.
Aspect 9: The coating powder of any one or more of of the preceding aspects, further comprising an additive, wherein the additive is a flow control agent, dry flow agent, antioxidant, pigment, dye, optical brightener, extender, heat stabilizer, light stabilizer, ultraviolet light stabilizer, ultraviolet light-absorbing compound, near infrared light-absorbing compound, infrared light-absorbing compound, plasticizer, lubricant, antistatic agent, anti-fog agent, antimicrobial agent, radiation stabilizer, flame retardant, anti-drip agent, fragrance, or a or a combination thereof.
Aspect 10: The coating powder of any one or more of the preceding aspects, wherein the particles have an average particle size of 10 to 100 micrometers.
Aspect 11: A method of forming the coating powder of any one or more of the preceding claims, comprising melt-blending the diepoxy phthalimidine, the curing agent, and optionally the auxiliary epoxy component; and forming a powder from the melt blend.
Aspect 12: A method of coating an article, the method comprising applying the coating powder of any one or more of claims 1 to 9 to a surface of the article; and heating the applied coating powder to melt and fuse the particles to form a film and cure the film to form a powder coating.
Aspect 13: The method of aspect 12, wherein the heating is at a temperature from 100 to 300°C, preferably 150 to 250°C; and for a time of up to 20 minutes, preferably up to 10 minutes, more preferably up to 5 minutes.
Aspect 14: An article comprising the powder coating of aspect 12 or aspect 13.
Aspect 15: The article of aspect 1, wherein the cured coating powder has at least one of a glass transition temperature greater than 120°C, greater than 140°C, preferably greater than 150°C, more preferably greater than 180°C, most preferably greater than 190°C; a glass transition temperature higher than a powder coating of the same composition but with a bisphenol A-based epoxy instead of the diepoxy phthalimidine; a hardness greater than a powder coating of the same composition but with a bisphenol A-based epoxy instead of the diepoxy phthalimidine; a ΔE color difference value of less than or equal to 1.0 after 252 kJ/m² of exposure, preferably less than or equal to 0.7 after 252 kJ/m² of exposure, more preferably 0.5 after 252 kJ/m² of exposure as determined according to ASTM 2244; and a gloss of 75 to 120, preferably 80 to 110 as measured according to ASTM D-523.

The compositions, methods, and articles disclosed herein can alternatively comprise, consist of, or consist essentially of, any appropriate components or steps herein disclosed. The compositions, methods, and articles can additionally, or alternatively, be formulated so as to be devoid, or substantially free, of any steps, components, materials, ingredients, adjuvants, or species that are otherwise not necessary to the achievement of the function and/or objectives of the compositions, methods, and articles.

All ranges disclosed herein are inclusive of the endpoints, and the endpoints are independently combinable with each other. "Combinations" is inclusive of blends, mixtures, alloys, reaction products, and the like. The terms "a" and "an" and "the" do not denote a limitation of quantity, and are to be construed to cover both the singular and the plural, unless otherwise indicated herein or clearly contradicted by context. "Or" means "and/or" unless clearly stated otherwise. The term "a combination thereof' includes one or more of the listed element, and is open to one or more like elements not listed. The described elements may be combined in any suitable manner in the various embodiments.

Compounds are described using standard nomenclature. For example, any position not substituted by any indicated group is understood to have its valency filled by a bond as indicated, or a hydrogen atom. A dash ("-") that is not between two letters or symbols is used to indicate a point of attachment for a substituent. For example, -CHO is attached through carbon of the carbonyl group. The term "alkyl" means a branched or straight chain, unsaturated aliphatic hydrocarbon group, e.g., methyl, ethyl, n-propyl, i-propyl, n-butyl, s-butyl, t-butyl, n-pentyl, s-pentyl, and n- and s-hexyl. "Alkenyl" means a straight or branched chain, monovalent hydrocarbon group having at least one carbon-carbon double bond (e.g., ethenyl (-HC=CH₂)). "Alkoxy" means an alkyl group that is linked via an oxygen (i.e., alkyl-O-), for example methoxy, ethoxy, and sec-butyloxy groups. "Alkylene" means a straight or branched chain, saturated, divalent aliphatic hydrocarbon group (e.g., methylene (-CH₂-) or, propylene (-(CH₂)₃-). "Cycloalkyl" means a divalent cyclic alkyl group, -CₙH₂ₙ₋ₓ, wherein x is the number of hydrogens replaced by cyclization(s). "Alkylarylene" means an arylene group substituted with an alkyl group. "Arylalkylene" means an alkylene group substituted with an aryl group (e.g., benzyl).

Unless substituents are otherwise specifically indicated, each of the foregoing groups can be unsubstituted or substituted, provided that the substitution does not significantly adversely affect synthesis, stability, or use of the compound. "Substituted" means that the compound, group, or atom is substituted with at least one (e.g., 1, 2, 3, or 4) substituents instead of hydrogen, where each substituent is independently nitro (-NO₂), cyano (-CN), hydroxy (-OH), halogen, thiol (-SH), thiocyano (-SCN), C₁₋₆ alkyl, C₂₋₆ alkenyl, C₂₋₆ alkynyl, C₁₋₆ haloalkyl, C₁₋₉ alkoxy, C₁₋₆ haloalkoxy, C₃₋₁₂ cycloalkyl, C₅₋₁₈ cycloalkenyl, C₆₋₁₂ aryl, C₇₋₁₃ arylalkylene (e.g., benzyl), C₇₋₁₂ alkylarylene (e.g, toluyl), C₄₋₁₂ heterocycloalkyl, C₃₋₁₂ heteroaryl, C₁₋₆ alkyl sulfonyl (-S(=O)₂-alkyl), C₆₋₁₂ arylsulfonyl (-S(=O)₂-aryl), or tosyl (CH₃C₆H₄SO₂-), provided that the substituted atom's normal valence is not exceeded, and that the substitution does not significantly adversely affect the manufacture, stability, or desired property of the compound. When a compound is substituted, the indicated number of carbon atoms is the total number of carbon atoms in the compound or group, including those of any substituents.

Unless specified to the contrary herein, all test standards are the most recent standard in effect as of the filing date of this application, or, if priority is claimed, the filing date of the earliest priority application in which the test standard appears.

Unless defined otherwise, technical and scientific terms used herein have the same meaning as is commonly understood by one of skill in the art to which this invention belongs. All cited patents, patent applications, and other references are incorporated herein by reference in their entirety. While particular embodiments have been described, alternatives, modifications, variations, improvements, and substantial equivalents that are or may be presently unforeseen may arise to applicants or others skilled in the art. Accordingly, the appended claims as filed and as they may be amended are intended to embrace all such alternatives, modifications variations, improvements, and substantial equivalents.

## Claims

1. A curable powder coating, comprising particles comprising a diepoxy phthalimidine of the formula: 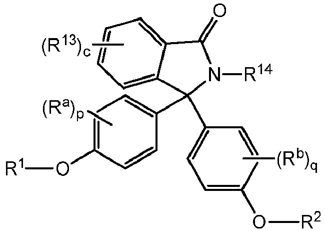 wherein
R¹ and R² are each independently an epoxide-containing functional group,
R^{a} and R^{b} at each occurrence are each independently halogen, C₁₋₁₂ alkyl, C₂₋₁₂ alkenyl, C₃₋₈ cycloalkyl, or C₁₋₁₂ alkoxy,
R¹³ at each occurrence is independently a halogen or a C₁-C₆ alkyl group,
p, q, and c are each independently 0 to 4, and
R¹⁴ is a C₁₋₆ alkyl or phenyl;
a curing agent; and
optionally, an auxiliary epoxy component co-curable with the diepoxy phthalimidine.

2. The coating powder of claim 1, wherein
R¹ and R² are each independently of the formula 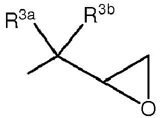
wherein R^{3a} and R^{3b} are each independently hydrogen or C₁₋₁₂ alkyl, preferably wherein R^{3a} and R^{3b} are each hydrogen,
R^{a} and R^{b} at each occurrence are each independently halogen, C₁₋₆ alkyl, C₂₋₆ alkenyl, C₃₋₆ cycloalkyl, or C₁₋₆ alkoxy, preferably halogen, C₁₋₆ alkyl, C₂₋₆ alkenyl, C₃₋₆ cycloalkyl, or C₁₋₆ alkoxy,
R¹³ at each occurrence is independently a halogen or a C₁₋₃ alkyl group, preferably a C₁₋₂ alkyl group,
R¹⁴ is C₁₋₂ alkyl or phenyl, and
p, q, and c are each independently 0 to 2.

3. The coating powder of claim 1, wherein
R¹ and R² at each occurrence are each independently 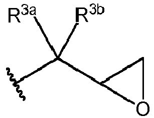
wherein R^{3a} and R^{3b} are each independently hydrogen or C₁₋₁₂ alkyl, preferably hydrogen;
R¹⁴ is C₁₋₂ alkyl or phenyl, , preferably methyl or phenyl, and
c, p, and q are each 0.

4. The coating powder of any one or more of the preceding claims, wherein the diepoxy phthalimidine is of the formula 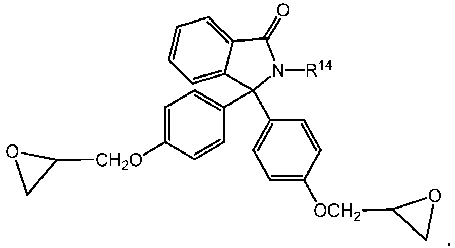 wherein R¹⁴ is methyl or phenyl, preferably wherein R¹⁴ is phenyl.

5. The coating powder of any one or more of the preceding claims, wherein the diepoxy phthalimidine has
a purity of greater than 60%, preferably greater than 70%, preferably greater than 80%, preferably greater than 90%, preferably greater than 95%, preferably greater than 98%, or preferably greater than 99% as determined by high performance liquid chromatography, or
an epoxy equivalent weight of 1.0 to 1.5, preferably 1.0 to 1.2, preferably 1.0 to 1.15, preferably 1.0 to 1.1, relative to the theoretical epoxy equivalent weight, or
a combination of the purity and epoxy equivalent weight.

6. The coating powder of any one or more of the preceding claims,
wherein the curing agent is an aliphatic amine, a cycloaliphatic amine, an aromatic amine, an amine-functional adduct with an epoxy resin, a Mannich bases, a polyamide, an amidoamine, a phenalkamine, a dicyandiamide, a polycarboxylic acid-functional polyester, a carboxylic acid anhydride an, amine-formaldehyde resin, a phenol-formaldehyde resin, a polysulfides, a polymercaptan, an imidazole, an imidazole adduct, a Lewis acid, a diaryliodonium salt, or a combination thereof;
preferably wherein the curing agent is an aromatic amine, an aromatic dianhydride, a bicyclic anhydride, dicyandiamide, a dicyandiamide adduct, an imidazole, an imidazole adduct, or a combination thereof;
preferably wherein the curing agent is 4,4'-diaminodiphenylmethane, 4,4'-diaminodiphenylsulfone, m-phenylenediamine, methyl-5-norbornene-2,3-dicarboxylic anhydride, dicyandiamide, an amide adduct of dicyandiamide, imidazole, 2-methyl imidazole, 4-methyl imidazole, 2,4-dimethyl imidazole, 2-methyl-4-ethyl imidazole, 2-ethyl-4-methyl imidazole, 2,4-diethyl imidazole, a dicyandiamide-imidazole adduct, an epoxy resin-imidazole adduct, or a combination thereof;
more preferably wherein the curing agent is 4,4'-diamino diphenyl sulfone , dicyandiamide, imidazole, 2-ethyl-4-methyl imidazole, a dicyandiamide-imidazole adduct, bisphenol A epoxy-imidazole adduct, or a combination thereof.

7. The coating powder of any one or more of the preceding claims, further comprising the auxiliary epoxy component co-curable with the diepoxy phthalimidine, wherein the auxiliary epoxy component is an aliphatic epoxy, cycloaliphatic epoxy, aromatic epoxy, multiaromatic epoxy, phenol novolac epoxy, cresol-novolac epoxy, biphenyl epoxy, triglycidyl p-aminophenol, tetraglycidyl diaminodiphenyl methane, naphthalene epoxy, divinylbenzene dioxide, 2-glycidylphenylglycidyl ether, dicyclopentadiene epoxy, or a combination thereof; preferably wherein the auxiliary component is a solid or semi-solid at 23°C.

8. The coating powder of any one or more of of the preceding claims, further comprising an additive, wherein the additive is a flow control agent, dry flow agent, antioxidant, pigment, dye, optical brightener, extender, heat stabilizer, light stabilizer, ultraviolet light stabilizer, ultraviolet light-absorbing compound, near infrared light-absorbing compound, infrared light-absorbing compound, plasticizer, lubricant, antistatic agent, anti-fog agent, antimicrobial agent, radiation stabilizer, flame retardant, anti-drip agent, fragrance, or a combination thereof.

9. The coating powder of any one or more of the preceding claims, wherein the particles have an average particle size of 10 to 100 micrometers.

10. A method of forming the coating powder of any one or more of the preceding claims, comprising
melt-blending the diepoxy phthalimidine, the curing agent, and optionally the auxiliary epoxy component; and
forming a powder from the melt blend.

11. A method of manufacture of a powder-coated article, the method comprising
applying the coating powder of any one or more of claims 1 to 9 to a surface of the article; and
heating the applied coating powder to melt and fuse the particles to form a film and cure the film to form a powder coating.

12. The method of claim 11, wherein the heating is at a temperature from 100 to 300°C, preferably 150 to 250°C; and for a time of up to 20 minutes, preferably up to 10 minutes, more preferably up to 5 minutes.

13. An article comprising the powder coating of claim 11 or claim 12.

14. The article of claim 1, wherein the powder coating has at least one of
a glass transition temperature greater than 120°C, greater than 140°C, greater than 150°C, greater than 180°C, preferably greater than 190°C;
a glass transition temperature greater than a powder coating of the same composition but with a bisphenol A-based epoxy instead of the diepoxy phthalimidine;
a hardness greater than a powder coating of the same composition but with a bisphenol A-based epoxy instead of the diepoxy phthalimidine;
a ΔE color difference value of less than or equal to 1.0 after 252 kJ/m² of exposure, preferably less than or equal to 0.7 after 252 kJ/m² of exposure, more preferably 0.5 after 252 kJ/m² of exposure as measured according to ASTM 2244; or
a gloss of 75 to 120, preferably 80 to 110, as measured according to ASTM D-523.
